Europäisches Patentamt

European Patent Office

Office européen des brevets

(19)

(11) **EP 1 236 512 A1**

(12) **EUROPEAN PATENT APPLICATION**

(43) Date of publication:
04.09.2002 Bulletin 2002/36

(51) Int Cl.⁷: **B01J 35/00**, B01J 31/06, B01J 13/00, C07C 29/17, C07C 33/035

(21) Application number: 01104866.7

(22) Date of filing: 28.02.2001

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **COUNCIL OF SCIENTIFIC AND INDUSTRIAL RESEARCH**
**New Delhi 100 001 (IN)**

(72) Inventors:
• **Rode, Chandrashekhar Vasant**
**Maharashtra (IN)**
• **Telkar, Manisha Madhukar**
**Maharashtra (IN)**
• **Chaudhari, Raghunath Vitthal**
**Maharashtra (IN)**

(74) Representative: **Viering, Jentschura & Partner**
**Postfach 22 14 43**
**80504 München (DE)**

(54) **Nanosized noble metal catalyst and process for selective preparation of 1,4 butenediol**

(57) This invention relates a nanosized noble metal catalyst for selective preparation of aliphatic diols having a general formula:

A/B

Wherein A is a polymeric colloiding agent and B a noble metal

and a process for the preparation of the said catalyst. The mole ratio of their composition (A/B) may be in the range of 1-100.
The catalyst is used in the selective hydrogeneration of 1,4 butynediol to 1,4 butenediol.

**EP 1 236 512 A1**

**Description**

**FIELD OF THE INVENTION**

This invention relates to a nanosized noble metal catalyst and preparation thereof. More particularly, it relates to the said catalyst composition in the form of a colloidal dispersion for selective preparation of 1,4 butenediol, and a process for the preparation of the said catalyst which is useful for selective hydrogenation of 1,4 butynediol to 1,4 butenediol. The catalyst is a noble metal e.g. palladium or platinum in the form of colloidal dispersion where the particle size of the catalysts is less than 75 nm.

**BACKGROUND OF THE INVENTION**

1,4 butenediol is a very useful intermediate in the production of pesticide, insecticide, and vitamin $B_6$. Being unsaturated diol it can be used in synthesis of many organic products such as tetrahydrofuran, n-methyl pyrrolidone, $\gamma$-butyrolactone etc. It is also used as an additive in paper industry, as a stabilizer in resin manufacture, as a lubricant for bearing system and in the synthesis of allyl phosphates.

**PRIOR ART REFERENCES**

In the prior art, the uses of a number of catalysts are described for producing 1,4 butenediol by hydrogenation of 1,4 butynediol. Most of these patents are based on the combination of palladium with one or more mixed compounds of copper, zinc, calcium, cadmium, lead, alumina, mercury, tellurium, gallium etc. Selective hydrogenation of acetylinic compound is described in GBA 871,804 in a suspension method using Pd catalyst which has been treated with the salt solutions of Zn, Cd, Hg, Ga, Th, In, or Ga. The process is carried out at milder conditions with 97% selectivity for cis 1,2-butenediol and 3% to the trans form. Moreover, use of organic amines has been suggested as promoters in the catalyst system.

Lindlar catalyst (lead doped Pd catalyst) is also used for the selective hydrogenation of the acetylinic compounds as described in US Patent No. 2,681,938. The drawback of this process is use of additional amines such as pyridine to obtain good selectivity for 1,4 butenediol.

Yet another DE patent (DE 1,213,839) describes Pd catalyst doped with Zn salts and ammonia for the partial hydrogenation of acetylinic compounds. But this catalyst suffers from a major drawback of short lifetime due to poisoning.

The use of $Pd/Al_2O_3$ catalyst, which has been treated with carbon monoxide for the hydrogenation of 1,4 butynediol in an inert solvent, is described in DE-A 2,619,660. The disadvantage of this process is that the catalyst is treated with carbon monoxide gas, which is highly toxic and difficult to handle.

US Patent No.2, 961,471 describes Raney nickel catalyst for partial hydrogenation of 1,4 butynediol using Raney nickel catalyst. This catalyst gave a low selectivity for 1,4 butenediol. In another, US Patent No. 2,953,604 is described a Pd containing charcoal and copper catalyst for the reduction of 1,4 butynediol to 1,4 butenediol with 81% selectivity for 1,4 butenediol. The drawback of the process is the formation of large amount of side products. Another US Patent No. 4,001,344 describes a catalyst for the preparation of 1,4 butenediol by hydrogenation of 1,4 butynediol by use of palladium mixed with $\gamma$-$Al_2O_3$ along with, zinc and cadmium or either zinc or cadmium together with bismuth or tellurium. The disadvantage of the said process is the formation of-large amount of residues (7.5-12%) which lowers the selectivity of 1,4 butenediol to 88%.

In addition to these patents, US Patent Nos. 5,521,139 and 5,728,900 describe a catalyst and a process for the preparation of 1,4 butenediol by the hydrogenation of 1,4 butynediol over a Pd containing catalyst. They have used the fixed bed catalyst, which was prepared by applying Pd and Pb or Pd and Cd successively, by vapor deposition or sputtering, to metal gauze or a metal foil acting as a support. In this process also selectivity obtained for cis 1,4 butenediol is 98%. The disadvantage of these processes is that a trans 1,4 butenediol with residues are also obtained.

All the above said catalysts for the hydrogenation of 1,4 butynediol to 1,4 butenediol suffers from the disadvantages such as used promoters like organic amines. Their preparation becomes cumbersome and involves use of sophisticated equipment. All these catalysts reported do not give complete selectivity to the desired product 1,4 butenediol. The formation of side products and residues has also been reported which affect the efficiency of the process and the recovery of pure 1,4 butenediol is difficult. Also these catalysts suffer from short life due to fast deactivation.

**OBJECT OF THE INVENTION**

The main object of the present invention is to provide a novel nanosized noble metal catalyst for selective preparation of aliphatic diols.

Another object of the present invention is to provide a novel nanosized noble metal catalyst for selective hydrogenation of 1,4 butynediol to 1,4 butenediol.

Yet another object of the invention is to provide a process for the preparation of nanosized noble metal catalyst in a colloidal form, useful for selective hydrogenation of 1,4 butynediol to 1,4 butenediol, wherein the noble metal may palladium or platinum and the colloiding agent may be polyvinylpyrrolidone, polyvinylalcohol, polyethyleneamine, polyvinylether, polyethyleneglycol, more preferably polyvinylpyrrolidone at very specific preparation conditions.

Still another object is to prepare colloidal dispersion of above-mentioned catalyst having the particle size less than 50 nm.

One more object of the invention is to provide a process for the preparation of 1,4 butenediol from 1,4 butynediol by selective hydrogenation using the catalyst of the present invention.

## SUMMARY OF THE INVENTION

To meet the above objects, the present invention provides nanosized noble metal catalyst for selective preparation of aliphatic diols having a general formula :

A/B

Wherein A is a polymeric colloiding agent
and B is a noble metal

The mole ratio of their composition (A/B) may be in the range of 1 - 100, and a process for the preparation of the said catalyst.

## DETAILED DESCRIPTION OF THE INVENTION

The nanosized noble metal catalyst, provided in the present invention is prepared by protecting the noble metal with polymer without poisoning, at very specific preparation conditions. The prepared catalyst is useful for the selective hydrogenation of 1-4 butynediol to 1,4 butenediol at the temperature ranging between 30°C to 90°C and $H_2$ pressure between 200-700 psig. The process of the present invention achieves complete conversion of 1,4 butynediol with min 85% selectivity to Cis butenediol without any addition of promoter in the substrate solution.

Recovery of pure 1,4 butenediol is done by fractional vacuum distillation.

The catalyst prepared by this process of the present invention gives complete conversion of 1,4 butynediol with min. 85% selectivity to 1,4 butenediol, at milder reaction conditions without adding any promoter to the substrate solution. Moreover, the highest purity of 1,4 butenediol is obtained merely by fractional distillation of reaction crude.

Accordingly, the present invention provides nanosized noble metal catalyst for selective preparation of aliphatic diols having a general formula

A/B

Wherein A is a polymeric colloiding agent
and B is a noble metal

The mole ratio of their composition (A/B) may be in the range of 1- 100 and a process for the preparation of the said catalyst which comprises, dissolving the noble metal precursor in a mixture of water and organic solvent, adding to this a solution of polymeric colloiding agent, adjusting the pH of the solution to about 4 by adding an alkali solution, refluxing the solution for 3 to 4 hrs and cooling to obtain the product.

In an embodiment of the present invention, the noble metal may be palladium or platinum

In another embodiment of the present invention, the mole ratio of A/B is in the range 1-60.

In another embodiment of the present invention, the size of the catalyst is in the range of 10-100nm.

In another embodiment of the present invention, the source of palladium or platinum is selected from the group consisting of salts of acetate, bromide and chloride, preferably $H_2PdCl_4$ for palladium and $H_2PtCl_6$ for platinum.

In yet another embodiment of the present invention, the colloidal agent may be selected from the group consisting of polyvinylpyrrolidone, polyvinylalcohol, polyethyleneamine, polyvinylether, polyethyleneglycol, more preferably poly vinyl pyrrolidone.

In yet another embodiment of the present invention, the organic solvent is selected from the group consisting of methanol, ethanol, isopropanol, 1,4 dioxane and dimethyl ether.

In yet another embodiment the mixture of water and organic solvent selected from the group consisting of methanol, ethanol, isopropanol, 1,4-dioxane and dimethyl ether is in the ratio of 1-100.

In further embodiment the present invention provides an improved process for preparation of 1,4 butenediol from 1,4 butynediol which comprises hydrogenating aqueous solution of 1,4 butynediol under stirring conditions, using nanosized noble metal catalyst, at a temperature ranging between 20-120°C under hydrogen atmosphere, cooling the reaction mixture to room temperature, separating the catalyst by conventional methods to obtain 1,4 butenediol.

In yet another embodiment, the concentration of 1,4 butynediol in aqueous medium is in the range of 10-70%.

In yet another embodiment, the hydrogen pressure may be in the range of 100-1200 psig.

In still another embodiment, the temperature of the reaction mixture may be in the range of 20-110°C.

The present process gives complete conversion of 1,4 butynediol with min 85% selectivity to 1,4 butenediol, at milder conditions without addition of additives or promoter in the substrate solution.

One embodiment of the present invention provides a process for the preparation of nanosized noble metal catalyst, prepared by protecting the noble metal with polymer without poisoning at very specific preparation conditions. The prepared catalyst is useful for the selective hydrogenation of 1,4 butynediol to 1,4 butenediol at the temperature ranging between 30°C to 90°C and hydrogen pressure between 200 to 700 psig. The present process achieves complete conversion of 1,4 butynediol with selectivity ranging between 85 to 100 % to cis 1,4 butenediol without any addition of promoter in the substrate solution. The catalyst activity, expressed in terms of TOF (hr[-1]) is nearly 300 times higher than reported in the literature. Moreover, in the highest purity 1,4 butenediol is obtained from the reaction mixture merely by fractional distillation.

The hydrogenation of 1,4 butynediol to 1,4 butenediol was carried out in an autoclave under stirring conditions in presence of a Pd containing catalysts suspended in a mixture of 1,4 butynediol in water without addition of ammonia at a temperature and $H_2$ pressure which is mentioned in the examples. Before pressurizing an autoclave, ensure that no air is present inside the autoclave. The hydrogenation is complete when the absorption of hydrogen is stopped or unchanged. After the reaction was complete, the reactor was cooled below ambient temperature, the contents were discharged, and reaction mixture was analyzed by gas chromatograph.

The process of the invention is described in detail in the examples given below which are illustrative only and should not be considered to limit the scope of the invention.

**EXAMPLE-1**

This example illustrates the preparation of nanosized Pd catalyst having a particle size 68nm, wherein the mole ratio of polyvinyl pyrolidone (PVP): Pd is 1:1 by the following procedure. 35.5 mg of palladium chloride was dissolved in 5 ml of concentrated hydrochloric acid and was diluted to 100 ml by adding water give 2.0 mmolar $H_2PdCl_4$. From this solution, 15 ml of 2.0 mmolar $H_2PdCl_4$ was withdrawn and 15 ml of ethyl alcohol was added. The pH of the solution was increased to 4 by adding 10% NaOH solution. To this solution 3.33 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

EXAMPLE-2

This example illustrates the preparation of nanosized Pd catalyst (particle size 44 nm) wherein the mole ratio of PVP: Pd is 10:1 by the following procedure. 35.5 mg of palladium chloride was dissolved in 5 ml of concentrated hydrochloric acid and was diluted to 100 ml by adding water to give 2.0 mmolar $H_2PdCl_4$. From this solution, 15 ml of 2.0 mmolar $H_2PdCl_4$ was withdrawn and 15 ml of ethyl alcohol was added. The pH of the solution was increased to 4 by adding 10% NaOH solution. To this solution 33.3 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

**EXAMPLE-3**

This example illustrates the preparation of nanosized Pd catalyst having a particle size 30nm, wherein the mole ratio of PVP: Pd is 20:1 by the following procedure. 355 mg of palladium chloride was dissolved in 5 ml of concentrated hydrochloric acid and was diluted to 100 ml by adding water to give 2.0 mmolar $H_2PdCl_4$. From this solution, 15 ml of 2.0 mmolar $H_2PdCl_4$ was withdrawn and 15 ml of ethyl alcohol was added. The pH of the solution was increased to 4 by adding 10% NaOH solution. To this solution 66.6 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

EXAMPLE-4

This example illustrates the preparation of nanosized (particle size 25 nm) Pd catalyst wherein the mole ratio of PVP: Pd is 30:1 by the following procedure. 35.5 mg of palladium chloride was dissolved in 5 ml of concentrated hydrochloric acid and was diluted to 100 ml by adding water to give 2.0 mmolar $H_2PdCl_4$. From this solution, 15 ml of 2.0 mmolar $H_2PdCl_4$ was withdrawn and 15 ml of ethyl alcohol was added. The pH of the solution was increased to 4 by adding 10% NaOH solution. To this solution 99.9 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

**EXAMPLE 5**

This example illustrates the preparation of nanosized (particle size 22nm) Pd catalyst wherein the mole ratio of PVP: Pd is 40:1 and the water to organic solvent ratio is 30:70 by the following procedure. 35.5 mg of palladium chloride was dissolved in 5 ml of concentrated hydrochloric acid and was diluted to 100 ml by adding water to give 2.0 mmolar $H_2PdCl_4$. From this solution, 15 ml of 2.0 mmolar $H_2PdCl_4$ was withdrawn and 15 ml of ethyl alcohol was added. The pH of the solution was increased to 4 by adding 10% NaOH solution. To this solution 133 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

EXAMPLE 6

This example illustrates the preparation of nanosized (particle size 22nm) Pd catalyst wherein the mole ratio of PVP: Pd is 40:1 and the water to organic solvent ratio is 50:50 by the following procedure. 67.4 mg of $PtCl_2$ was dissolved in 5 ml of concentrated hydrochloric acid and was diluted to 100 ml by adding water to give 2.0 mmolar solution of $H_2PtCl_4$. From this solution, 15 ml of 2.0 mmolar $H_2PtCl_4$ was withdrawn and 25 ml of ethyl alcohol was added. The pH of the solution was increased to 4 by adding 10% NaOH solution. To this solution 133 mg of PVP was added, then the solution was diluted to 50 ml by adding water and the mixture was refluxed for 3 hrs. After 3 hrs. the solution was cooled and directly used for hydrogenation of 1,4 butynediol to 1,4 butenediol.

EXAMPLE 7

This example illustrates the preparation of nanosized (particle size 20nm) Pt catalyst wherein the mole ratio of PVP: Pt is 40:1 and the water to organic solvent ratio is 30:70 by the following procedure. 67.4 mg of $PtCl_4$ was dissolved in.5 ml of concentrated hydrochloric acid and was diluted to 100 ml by adding water to give 2.0 mmolar solution of $H_2PtCl_4$. From this solution, 15 ml of 2.0 mmolar $H_2PtCl_4$ was withdrawn and 15 ml of ethyl alcohol was added. The pH of the solution was increased to 4 by adding 10% NaOH solution. To this solution 133 mg of PVP was added, then the solution was diluted to 50 ml by adding water and the mixture was refluxed for 3 hrs. After 3 hrs the solution was cooled and directly used for hydrogenation of 1,4 butynediol to 1,4 butenediol.

EXAMPLE-8

This example illustrates the performance of the nanosized palladium and platinum catalysts as described in examples 1-7 for the hydrogenation of 1,4 butynediol to 1,4 butenediol as follows:

Conc. of 1,4 butynediol (Aq.) :   20%
Conc. of catalyst :   0.5 ml
Temperature :   50°C
$H_2$ pressure :   350 psig

| Example No | Catalyst | Particle size (nm) | Water : Ethanol | TOF x $10^{-5}$ ($hr^{-1}$) | Selectivity to 1,4 butenediol, (%) |
|---|---|---|---|---|---|
| 1. | PVP/Pd=1 | 68 | 30:70 | 2.0 | 94.6 |
| 2. | PVP/Pd=10 | 44 | 30:70 | 2.2 | 96.2 |
| 3. | PVP/Pd=20 | 30 | 30:70 | 2.8 | 93.8 |

(continued)

| Example No | Catalyst | Particle size (nm) | Water : Ethanol | TOF x $10^{-5}$ (hr$^{-1}$) | Selectivity to 1,4 butenediol, (%) |
|---|---|---|---|---|---|
| 4. | PVP/Pd=30 | 25 | 30:70 | 3.0 | 87.7 |
| 5. | PVP/Pd=40 | 22 | 30:70 | 3.5 | 100 |
| 6. | PVP/Pd=40 | 22 | 50:50 | 2.5 | 100 |
| 7. | PVP/Pt=40 | 20 | 30:70 | 2.6 | 100 |

The advantages of the present invention are:

1. Selective hydrogenation of 1,4 butynediol to 1,4 butenediol is achieved by using nanosized noble metal catalysts without poisoning it.
2. The particle size of the catalyst is below 50 nm.
3. Selective preparation of 1,4 butenediol is achieved without any additive or promoter in the substrate solution.
4. No special reduction method for catalyst is needed; the solvent itself reduces the catalyst.
5. Results obtained under milder reaction conditions.
6. Turn over number obtained is in the range of 2 - 3.5 x $10^6$ which, is higher than that reported in the literature.
7. Recovery of pure 1,4 butenediol is done by fractional vacuum distillation.

## Claims

1. A novel nanosized noble metal catalyst for selective preparation of aliphatic diols having a general formula

A/B

Wherein A is a polymeric colloiding agent
and B is a noble metal;
the mole ratio of their composition may be in the range of 1 - 100.

2. A catalyst as claimed in claim 1 wherein, the polymeric colloiding agent is selected from polyvinylpyrrolidone, polyvinylalcohol, polyethyleneamine, polyvinylether, polyethyleneglycol, and more preferably polyvinylpyrrolidone.

3. A catalyst as claimed in claim 1 wherein, the noble metal is palladium or platinum.

4. A catalyst as claimed in claim 1 wherein, the size of the catalyst is in the range of 10-100 nm.

5. A catalyst as claimed in claim 1 wherein, the source of palladium or platinum is selected from the group consisting of Palladium or Platinum salts of acetate, bromide and chloride, preferably $H_2PdCl_4$ for palladium and $H_2PtCl_6$ for platinum.

6. A process for preparing a nanosized noble metal catalyst as claimed in claim 1, the said process comprising

a. dissolving a noble metal precursor in a mixture of water and organic solvent,
b. adding to this solution a polymeric colloiding agent,
c. adjusting the pH of the solution to about 4 by adding an alkali solution, and
d. refluxing the solution for 3 to 4 hrs and cooling to obtain the product.

7. A process as claimed in claim 6 wherein, the noble metal is a palladium or platinum.

8. A process as claimed in claim 6 wherein, the size of the catalyst is in the range of 10-100 nm.

9. A process as claimed in claim 6 wherein, the source of palladium or platinum is selected from the group consisting of Palladium or Platinum salts of acetate, bromide and chloride, preferably $H_2PdCl_4$ for palladium and $H_2PtCl_6$ for

platinum.

10. A process as claimed in claim 6 wherein, the colloidal agent is selected from the group consisting of polyvinylpyrrolidone, polyvinylalcohol, polyethyleneamine, polyvinylether, polyethyleneglycol, and more preferably polyvinylpyrrolidone.

11. A process as claimed in claim 6 wherein, the organic solvent is selected from methanol, ethanol, isopropanol, 1,4-dioxane and dimethyl ether.

12. A process as claimed in claim 6 wherein, a mixture of water and organic solvent selected from the group consisting of methanol, ethanol, isopropanol, 1,4-dioxane and dimethyl ether is in the ratio of 1-100.

13. A process for the preparation of 1,4 butenediol from 1,4 butynediol, which comprises hydrogenating aqueous solution of 1,4 butynediol under stirring conditions, using a nanosized noble metal catalyst as defined in claim 1, at a temperature ranging between 20-120°C under hydrogen atmosphere, cooling the reaction mixture to room temperature, separating tile catalyst by conventional methods to obtain 1,4 butenediol.

14. A process as claimed in claim 13 wherein, the concentration of 1,4 butynediol in aqueous medium is in the range of 10-70%.

15. A process as claimed in claims 13 wherein, the hydrogen pressure is in range of 100-1200 psig.

16. A process as claimed in claim 13 wherein, the temperature of the reaction is in the range of 20-110°C.

17. A process as claimed in claim 13 which provides the complete conversion of 1,4 butynediol with selectivity ranging between 85 - 100% to 1,4 butenediol, at milder conditions without addition of additives or promoter in the substrate solution.

EP 1 236 512 A1

## European Patent Office

## EUROPEAN SEARCH REPORT

**Application Number**

EP 01 10 4866

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | HIRAI H ET AL: "PREPARATION OF COLLOIDAL TRANSITION METALS IN POLYMERS BY REDUCTION WITH ALCOHOLS OR ETHERS" JOURNAL OF MACROMOLECULAR SCIENCE: PART A – CHEMISTRY, MARCEL DEKKER, NEW YORK, NY, US, vol. 13, no. 6, 1979, pages 727-750, XP000943145 ISSN: 0022-233X | 1-5 | B01J35/00 B01J31/06 B01J13/00 C07C29/17 C07C33/035 |
| Y | * the whole document * | 6-12 | |
| X | DE 195 00 366 C (BASF AG) 2 May 1996 (1996-05-02) * column 3, line 11 – line 37; claim 1 * | 1-5 | |
| Y | * examples 1,2,4,7 * | 6-12 | |
| Y | * column 1, line 13-41 * * column 4, line 21-35 * | 13-17 | |
| X | YUAN WANG ET AL: "IMMOBILIZATION OF POLYMER-PROTECTED METAL COLLOID CATALYSTS BY THE FORMATION OF POLYMER HYDROGEN BOND COMPLEXES" POLYMERS FOR ADVANCED TECHNOLOGIES, JOHN WILEY AND SONS, CHICHESTER, GB, vol. 7, no. 8, 1 August 1996 (1996-08-01), pages 634-638, XP000623415 ISSN: 1042-7147 * page 634 – page 635; table 1 * | 1-5 | **TECHNICAL FIELDS SEARCHED (Int.Cl.7)** B01J C07C |
| Y | * page 635, column 1, paragraph 3 * | 6-12 | |
| Y | * page 636, column 2, paragraph 2 – page 637, column 2, paragraph 1; tables 1,3 * | 13-17 | |
| X | US 5 929 261 A (SIGWART CHRISTOPH ET AL) 27 July 1999 (1999-07-27) * column 3, line 18 – line 34 * * claims 1-3 * | 1-5 | |
| Y | * examples 3,5-9 * | 6-12 | |
| A | * column 2, line 55 – column 3, line 3 * | 13 | |

-/--

The present search report has been drawn up for all claims

| Place of search | Date of completion of the search | Examiner |
|---|---|---|
| MUNICH | 21 December 2001 | Goebel, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

8

**European Patent Office**

## EUROPEAN SEARCH REPORT

Application Number

EP 01 10 4866

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 198 03 891 A (BAYER AG) 5 August 1999 (1999-08-05) * column 3, line 67 - column 4, line 11; claims 1,5 * | 1-5 | |
| Y | * column 3, line 17 - line 32 * * column 3, line 62 - line 67 * * claims 2,9; examples 1,2 * | 6-12 | |
| X | DE 44 10 353 A (YUKONG LTD) 29 September 1994 (1994-09-29) * page 3, line 35 - line 37; claims 9-11 * | 1-3,5 | |
| Y | * page 3, line 13 - line 18; claim 12; examples A,B,D * | 6-12 | |
| X | WO 00 55165 A (HAEHNLEIN MARC SASCHA ;VORLOP KLAUS DIETER (DE); CAPAN EMINE (DE);) 21 September 2000 (2000-09-21) * page 12, paragraph 3 - page 13, paragraph 2 * * page 9, paragraph 5 - page 10, paragraph 1 * * claims 1,8,11 * | 1-5 | |
| Y | * example 1 * | 6-12 | TECHNICAL FIELDS SEARCHED (Int.Cl.7) |
| X | US 4 725 314 A (GULLA MICHAEL ET AL) 16 February 1988 (1988-02-16) * examples 1-13 * * claims 1,4,5,12-16 * | 1-5 | |
| Y | * column 7, line 10 - column 8, line 41 * | 6-12 | |
| X | US 5 514 602 A (BROOKS JR HOUSTON G ET AL) 7 May 1996 (1996-05-07) * column 4, line 18 - line 21; claim 5; example 1 * | 1,2,4 | |
| Y | * column 5, line 24 - line 38 * * column 5, line 65 - column 6, line 10 * * claims 1-3 * | 6,8,10 | |

-/--

The present search report has been drawn up for all claims

| Place of search | Date of completion of the search | Examiner |
|---|---|---|
| MUNICH | 21 December 2001 | Goebel, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

## EUROPEAN SEARCH REPORT

Application Number

EP 01 10 4866

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 665 266 A (BASF AG) . 2 August 1995 (1995-08-02) * column 1, line 1 - line 9 * * column 1, line 52 - column 2, line 37 * * examples 1,2 * | 1-5 | |
| X | WO 01 10551 A (EASTHAM GRAHAM RONALD ;INEOS ACRYLICS UK LTD (GB); JOHNSON DAVID W) 15 February 2001 (2001-02-15) * page 3, paragraph 3 * * page 6, paragraph 6 * * examples 1-6; table 1 * orig. claims 1,5,9 & amended claims 1,3,7 | 1-5 | |
| X | DE 196 20 171 A (NIGU CHEMIE GMBH) 27 November 1997 (1997-11-27) * column 2, line 37 - line 42; claims; examples * | 1-5 | |
| A | * column 1, line 51-58 * | 13 | |
| X | DE 197 54 304 A (HOECHST AG) 10 June 1999 (1999-06-10) * page 5, line 18 - line 19 * * claims 1,10 * | 1,3,5 | TECHNICAL FIELDS SEARCHED (Int.Cl.7) |
| Y,D | GB 871 804 A (BASF AG) 28 June 1961 (1961-06-28) * page 1, line 51-75 * * page 2, line 91-95 * * examples 2,10,14,15 * | 13-17 | |
| A | US 5 849 652 A (BALKUS JR KENNETH J ET AL) 15 December 1998 (1998-12-15) * column 9, line 38-47; example III; table III * | 13-17 | |

The present search report has been drawn up for all claims

| Place of search | Date of completion of the search | Examiner |
|---|---|---|
| MUNICH | 21 December 2001 | Goebel, M |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**                    EP 01 10 4866

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2001

| Patent document cited in search report | | Publication date | Patent family member(s) | | Publication date |
|---|---|---|---|---|---|
| DE 19500366 | C | 02-05-1996 | US | 5753583 A | 19-05-1998 |
| | | | DE | 19500366 C1 | 02-05-1996 |
| | | | DE | 59500603 D1 | 09-10-1997 |
| | | | EP | 0724907 A2 | 07-08-1996 |
| | | | JP | 8229392 A | 10-09-1996 |
| US 5929261 | A | 27-07-1999 | DE | 19602710 A1 | 31-07-1997 |
| | | | CA | 2243931 A1 | 31-07-1997 |
| | | | CN | 1209806 A | 03-03-1999 |
| | | | DE | 59700697 D1 | 16-12-1999 |
| | | | WO | 9727182 A1 | 31-07-1997 |
| | | | EP | 0888321 A1 | 07-01-1999 |
| | | | ES | 2139438 T3 | 01-02-2000 |
| | | | JP | 2000503979 T | 04-04-2000 |
| DE 19803891 | A | 05-08-1999 | DE | 19803891 A1 | 05-08-1999 |
| | | | AU | 2619099 A | 16-08-1999 |
| | | | WO | 9938615 A1 | 05-08-1999 |
| DE 4410353 | A | 29-09-1994 | KR | 9605493 B1 | 25-04-1996 |
| | | | DE | 4410353 A1 | 29-09-1994 |
| | | | JP | 7100380 A | 18-04-1995 |
| WO 0055165 | A | 21-09-2000 | DE | 19911504 A1 | 19-10-2000 |
| | | | AU | 4395300 A | 04-10-2000 |
| | | | WO | 0055165 A1 | 21-09-2000 |
| US 4725314 | A | 16-02-1988 | US | 4634468 A | 06-01-1987 |
| | | | AU | 579531 B2 | 24-11-1988 |
| | | | AU | 4201585 A | 14-11-1985 |
| | | | CA | 1246535 A1 | 13-12-1988 |
| | | | CN | 85105647 A | 28-01-1987 |
| | | | EP | 0163831 A2 | 11-12-1985 |
| | | | JP | 61028450 A | 08-02-1986 |
| | | | US | 4652311 A | 24-03-1987 |
| US 5514602 | A | 07-05-1996 | US | 5571726 A | 05-11-1996 |
| | | | AU | 650632 B2 | 30-06-1994 |
| | | | AU | 6660690 A | 23-05-1991 |
| | | | AU | 7438094 A | 15-12-1994 |
| | | | CA | 2029942 A1 | 17-05-1991 |
| | | | EP | 0428412 A2 | 22-05-1991 |
| | | | GR | 90100804 A ,B | 17-04-1992 |
| | | | JP | 3209166 A | 12-09-1991 |
| | | | AT | 79956 T | 15-09-1992 |
| | | | AU | 602694 B2 | 25-10-1990 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 01 10 4866

21-12-2001

| Patent document cited in search report | | Publication date | Patent family member(s) | | Publication date |
|---|---|---|---|---|---|
| US 5514602 | A | | AU | 7385487 A | 10-12-1987 |
| | | | CA | 1288337 A1 | 03-09-1991 |
| | | | DE | 3781335 D1 | 01-10-1992 |
| | | | DE | 3781335 T2 | 17-12-1992 |
| | | | EP | 0250137 A2 | 23-12-1987 |
| | | | JP | 2537873 B2 | 25-09-1996 |
| | | | JP | 63025553 A | 03-02-1988 |
| | | | MX | 173476 B | 08-03-1994 |
| EP 0665266 | A | 02-08-1995 | DE | 4402890 A1 | 03-08-1995 |
| | | | EP | 0665266 A2 | 02-08-1995 |
| | | | JP | 8027307 A | 30-01-1996 |
| WO 0110551 | A | 15-02-2001 | AU | 6304500 A | 05-03-2001 |
| | | | WO | 0110551 A1 | 15-02-2001 |
| DE 19620171 | A | 27-11-1997 | DE | 19620171 A1 | 27-11-1997 |
| | | | AU | 2956797 A | 09-12-1997 |
| | | | WO | 9744304 A1 | 27-11-1997 |
| | | | EP | 0901455 A1 | 17-03-1999 |
| DE 19754304 | A | 10-06-1999 | DE | 19754304 A1 | 10-06-1999 |
| | | | WO | 9929423 A1 | 17-06-1999 |
| | | | EP | 1039968 A1 | 04-10-2000 |
| GB 871804 | A | 28-06-1961 | BE | 564339 A | |
| | | | CH | 367484 A | 28-02-1963 |
| | | | DE | 1115238 B | |
| | | | FR | 1190949 A | 15-10-1959 |
| | | | NL | 102759 C | |
| US 5849652 | A | 15-12-1998 | NONE | | |

EPO FORM P0459